# EUROPEAN PATENT APPLICATION

(11) **EP 3 095 425 A1**
(43) Date of publication of application: **23.11.2016**
(21) Application number: 15168935.3
(22) Date of filing: 22.05.2015
(51) Int. Cl.: A61F 13/84, G01N 19/00

(54) **METHOD FOR ASSESSING THE PERMANENCY OF CHANNELS IN AN ABSORBENT CORE**

(71) Applicant: The Procter & Gamble Company, Cincinnati, OH 45202 (US)
(72) Inventor: Baquer Molas, Gemma, 65824 Schwalbach am Taunus (DE); Le, Nguyen Huynh-Trang, 65824 Schwalbach am Taunus (DE); Wiesemann, Frank, 65824 Schwalbach am Taunus (DE)
(74) Representative: L'Huillier, Florent Charles

(57) **Abstract**

A method for evaluating the permanency of channels in an absorbent core, the method comprising the steps of: - providing an absorbent core (28), optionally in an absorbent article (20), wherein the absorbent layer comprises a least one channel (26), preferably at least two channels, extending in the longitudinal direction; - providing a liquid (40); - pouring the liquid into the absorbent core so that the absorbent core absorbs at least some of the liquid; optionally squeezing of shaking the absorbent core; - cutting the absorbent core in the transversal direction; and - evaluating the presence or absence of channel bonds (27), and if channel bonds are present optionally evaluating the quality of the channel bonds.

## Description

### FIELD OF THE INVENTION

The present invention relates to a method for evaluating certain properties of channels in an absorbent core. The absorbent core can be used in any personal hygiene absorbent articles such as baby and infant diapers, training pants or adult incontinence products. The method may be conducted on an isolated absorbent core or an absorbent article containing such an absorbent core. In particular, the method allows easily evaluating the permanency, or conversely the fugacity, of channels in an absorbent core in usage conditions.

### BACKGROUND OF THE INVENTION

Absorbent articles for personal hygiene such as diapers are designed to absorb and contain body exudates, in particular large quantity of urine. These absorbent articles comprise several layers, for example a wearer-facing topsheet, a garment-facing backsheet and in-between an absorbent core, among other layers. The function of the absorbent core is typically to absorb and retain the exudates for a prolonged amount of time, minimize re-wet to keep the wearer dry and avoid soiling of clothes or bed sheets.

The majority of absorbent cores comprise an absorbent material within a core wrap. A first type of commonly used absorbent material is a blend of comminuted wood pulp (so-called "air-felt") with superabsorbent polymers (SAP) in particulate form, also called absorbent gelling materials (AGM). Another type of cores having SAP as absorbent material without cellulose fibers (so called "airfelt-free" cores) has also been more recently proposed. Fluid-distributing channels extending longitudinally have been proposed for both types of cores. The channels can distribute an insulting fluid quickly along a greater area of the absorbent core thus improving fluid acquisition and optimizing absorbent material usage. Channels may also be used to facilitate the folding of the absorbent core in a pre-determined fashion, thus improving the anatomical conformity of the article. Various channel designs have thus been suggested. In air-felt cores, channels may be provided for example by locally embossing the absorbent material. Channels may also be provided by zones substantially free of absorbent material and surrounded by absorbent material. The top side of core wrap may be attached to the bottom side of the core wrap through these areas substantially free of absorbent material by a core wrap bond (herein "channel bond"), so that the channels are more resilient to the movement of the wearer or when the core is swelling with fluid. The core wrap typically comprises one or two layers of a nonwoven synthetic material, typically PP or PE. The channel bonds may be provided by various means such as gluing, pressure, heat and/or ultrasonic bonding of the core wrap. On the other hand, it is simpler and less costly for the manufacturer to not bond the core wrap through the channels.

It is typically beneficial that the channel bonds are sufficiently permanent to ensure the desired fluid-distributing or form-shaping properties of the core during the use of the product. Although it may be desirable that at a high load the channels bonds starts releasing so as to free more space for the absorbent material to swell, the channel bonds should remain present through the majority of the usage time of the article. Channels may be thus generally classified in three categories: permanent, semi-permanent and non-bonded. Permanent channel bonds are obtained by strongly bonding the core wrap through the material-free channel areas so that the channel bonds will not easily break, for example only break when a high core loading is achieved. Semi-permanent channel bonds will on the other hand be looser and may break or release at moderate loading and/or when the absorbent core is subjected to compression. Non-bonded channels that do not comprise any channel bonds will typically quickly be filled by the absorbent material as it swells and thus provide little functional benefits to the core.

Laboratory tests have been suggested to measure the permanency of channels. There is a however a need for simpler methods to visually evaluate and compare the permanency of channels used in absorbent cores.

### SUMMARY OF THE INVENTION

The method of the invention can be used for evaluating the permanency of fluid-distributing channels in an absorbent core. The method comprises the subsequent steps of:
- providing an absorbent core, optionally in an absorbent article, extending in a longitudinal direction and a transversal direction, wherein the absorbent core comprises a core wrap having a top layer and a bottom layer, and an absorbent layer comprising an absorbent material between the top layer of the core wrap and the bottom layer of the core wrap, and wherein the absorbent layer comprises a least one channel, preferably at least two channels, generally extending in the longitudinal direction;
- providing a liquid;
- pouring the liquid so that the absorbent layer absorbs at least some of the liquid;
- optionally exerting a physical action on the core such as squeezing or shaking the absorbent core;
- cutting the absorbent core in a transversal direction in an area of the core where the channel(s) is/are present; and
- visually evaluating the presence or absence of channel bonds between the top layer and the bottom layer through the cut in the core, and if channel bonds are present optionally evaluating the quality of the channel bonds.

The method is applicable to an absorbent core in isolation or to an absorbent article comprising such an absorbent core. The step of cutting the absorbent core/article may take place before or after the step of pouring the liquid. The method may comprise a step of physically acting on the core such as squeezing or shaking the absorbent core before the step of cutting the core. This simulates the compression exerted by a wearer when walking, sitting, jumping or doing other activities with the article on. The core may also be cut before the liquid is poured to better visualize the evolution of the channels while the absorbent material swells. More generally, the method steps are applicable to evaluate channels present between any layers of an article. The invention is also directed to a method for comparing different absorbent articles/cores, in particular where one product has channels and the other not, or both products have channels but one has stronger (more permanent) channels than the other. This and further aspects of the method of the invention are indicated in the claims, and are further described in the following description.

### BRIEF DESCRIPTION OF THE DRAWINGS

Fig. 1 shows a top view of an exemplary absorbent core comprising a pair of channels extending longitudinally;
Fig. 2a, b, c illustrate a cross-sectional view (not-to-scale) of three types of channels formed within the material-free areas: permanent, semi-permanent and non-bonded;
Fig. 3a, b, c, shows a cross-sectional view of the three absorbent cores of Figs. 2 after a moderate amount of liquid has been absorbed by the absorbent core;
Fig. 4a, b, c shows a cross-sectional view of the three absorbent cores of Fig. 3 after the core has been laterally compressed and/or squeezed;
Fig. 5 illustrates the step of providing an absorbent article in the form of a diaper and a liquid for conducting the method;
Fig. 6 illustrates the step of pouring some of the liquid in the middle of the absorbent article;
Fig. 7 illustrates the step of manually squeezing the absorbent article;
Fig. 8 illustrates the step of cutting transversally the swollen diaper;
Fig. 9 shows one of the diaper's half obtained after cutting the article transversally through its middle.

### DETAILED DESCRIPTION OF THE INVENTION

### Introduction

As used herein, the terms "comprise(s)" and "comprising" are open-ended; each specifies the presence of the feature that follows, e.g. a component, but does not preclude the presence of other features, e.g. elements, steps, components known in the art or disclosed herein. These terms based on the verb "comprise" should be read as encompassing the narrower terms "consisting essentially of" which excludes any element, step or ingredient not mentioned which materially affect the way the feature performs its function, and the term "consisting of" which excludes any element, step, or ingredient not specified. Any preferred or exemplary embodiments described below are not limiting the scope of the claims, unless specifically indicated to do so. The words "typically", "normally", "preferably", "advantageously", "in particular" and the likes also qualify features which are not intended to limit the scope of the claims unless specifically indicated to do so.

The invention will now be further illustrated with reference to the embodiments as described in the Figures. For ease of discussion, the absorbent core, absorbent article and their components will be discussed with reference to the numerals referred to in these Figures. However it should be understood that these exemplary embodiments and the numerals are not intended to limit the scope of the claims, unless specifically indicated. Dimensions and values disclosed herein are not to be understood as being strictly limited to the exact numerical values recited. Instead, unless otherwise specified, each such dimension is intended to mean both the recited value and a functionally equivalent range surrounding that value. For example, a dimension disclosed as "40 mm" is intended to mean "about 40 mm".

As used herein, the term "wearer" refers to the person, which may be an adult, child, or baby, that suffers from incontinence and actually wears the absorbent product in which the absorbent core is or will be incorporated. The term "user" refers to the caregiver that applies the absorbent article on the wearer. The "user" may be a parent, a family member in general or a professionally employed caregiver. The "tester" refers to the person conducting the method of the invention. The tester may be a prospective user or wearer, a professional tester working in a testing institute or at an absorbent article's manufacturer site, or an interested person desirous to compare different articles and share the results with the public (a blogger for example).

### General description of an absorbent core

As used herein, the term "absorbent core" refers to a component used or intended to be used in an absorbent article and which comprises an absorbent material enclosed in a core wrap. As used herein, the term "absorbent core" does not include the topsheet, the backsheet and (if present) any acquisition-distribution layer or multilayer system, which is not integral part of the absorbent core. The absorbent core is typically the component of an absorbent article that has the most absorbent capacity of all the components of the absorbent article, and which comprises all, or at least the majority of, superabsorbent polymer (SAP). The absorbent core may consist essentially of, or consist of, the core wrap, the absorbent material and optionally adhesives. The terms "absorbent core" and "core" are herein used interchangeably. The present method can be used on an absorbent core in isolation, or on a ready-to-use absorbent article comprising the absorbent core.

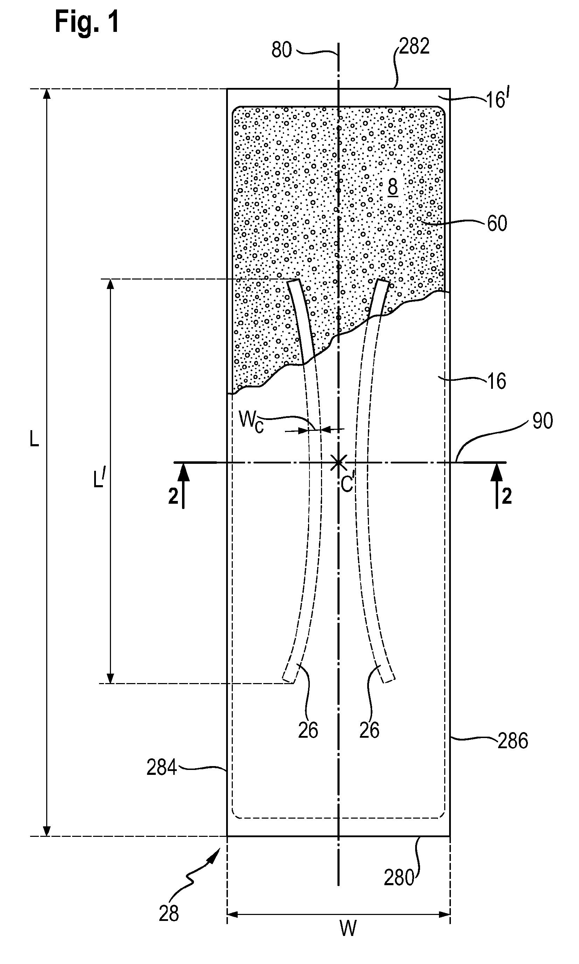

An exemplary core 28 is represented in Fig. 1 in a dry state outside an absorbent article. Absorbent cores can typically be laid flat on a surface as shown on Fig. 1. Absorbent cores may also be typically thin and conformable, so that they can also be laid on a non-flat surface for example a drum during their making process or stored as a continuous roll of stock material before being converted into an absorbent article. For ease of discussion, the exemplarily absorbent core of Fig. 1 is represented in a flat state and extending in a longitudinal direction 80 and a transversal direction 90. Unless otherwise indicated, dimensions and areas disclosed herein apply to the core in this flat-out configuration. The same applies to the absorbent article in which the core is integrated.

The absorbent core can typically be generally rectangular with a width W in the transversal direction and a length L in the longitudinal direction as measured from edge to edge, including the region of the core wrap which does not enclose the absorbent material, in particular at the front and back ends 280, 282, which may be or not be sealed. In case the core is not rectangular, the maximum dimension measured along the transversal and longitudinal direction can be used to report the length and width of the core. The width and length of the core may vary depending on the intended usage. For baby and infant diapers, the width W may for example in the range from 40 mm to 200 mm and the length L from 100 mm to 500 mm, as measured along the longitudinal axis 80 of the core. The longitudinal axis 80 of the core may be contiguous with the longitudinal axis of the article in which it is incorporated. The article further typically comprises a liquid permeable topsheet on the wearer-facing side and a liquid impermeable backsheet on the garment-facing side with the absorbent core positioned between the topsheet and the backsheet.

The core wrap may comprise a top layer 16 generally forming the top side of the core and a bottom layer 16' generally forming the bottom side of the core wrap as shown in the Figs. 1-2. The top and bottom layers may be formed by two separate substrates which may be the same or different material (the top layer being for example hydrophillically treated), but any other known core wrap constructions may also be used, for example wherein the core wrap is formed of a single web wrapped around the absorbent material with one single longitudinal seal. The top and bottom layer can be attached by gluing or otherwise to form at least one C-wrap seal along each of the longitudinally-extending side edges 284, 286 of the core. The material of the top and bottom layers may be a nonwoven web, such as a laminate comprising spunbond ("S") or meltblown ("M") layer. For example spunmelt polypropylene nonwovens are suitable, in particular those having a laminate web SMS, or SMMS, or SSMMS, structure, and having a basis weight range of about 5 gsm to 15 gsm. Suitable materials are for example disclosed in US 7,744,576, US 2011/0268932 A1, US 2011/0319848 A1 and US 2011/0250413 A1. The bottom layer 16' may be inherently hydrophobic but air-permeable, and the top layer 16 may be hydrophillically treated. There may be a seal along the front edge 282 and back edge 280 of the core wrap for better containment of the absorbent material but many cores do not have such transversal seals.

The absorbent material in the core can be of any type, in particular it can comprise wood pulp fibers mixed with superabsorbent polymers (herein abbreviated as "SAP") or be free of such cellulose fibers ("airfelt-free" core). The first type of core typically comprises from 40% to 80% of SAP. For absorbent cores comprising a relatively high proportion of superabsorbent polymer enclosed within the core wrap, the SAP content may represent in particular at least 85%, 90%, 95% and up to 100%, of superabsorbent polymer by weight of the absorbent material. The absorbent material may in particular comprise no or only small amount of cellulose fibers, such as less than 20%, in particular less than 10%, 5% or even 0% of cellulose fibers by weight of the absorbent material. The absorbent material may thus advantageously consist or consist essentially of SAP. The SAP may be typically in particulate forms (superabsorbent polymer particles), but it not excluded that other forms of SAP may be used such as a superabsorbent polymer foam for example. The absorbent core may thus be relatively thin, in particular thinner than conventional cores comprising cellulosic fibers.

The term "superabsorbent polymer" refers herein to absorbent material, which may be cross-linked polymer, and that can typically absorb at least 10 times their weight of an aqueous 0.9% saline solution as measured using the Centrifuge Retention Capacity (CRC) test (EDANA method WSP 241.2-05E). The SAP may in particular have a CRC value of more than 20 g/g, or more than 24 g/g, or of from 20 to 50 g/g, or from 20 to 40 g/g, or from 24 to 30 g/g.

The absorbent material 60 defines an absorbent material deposition area 8, as seen from above within the plane of the core. The absorbent material deposition area 8 is defined by the periphery of the layer of absorbent material 60 within the core wrap, as seen from the top side of the absorbent core as shown on Fig. 1, and comprises the channels 26 encompassed within. The absorbent material deposition area 8 can be generally rectangular, for example as shown in Fig. 1, but other shapes can also be used such as a "T" or "Y" or "sand-hour" or "dog-bone" shape. In particular the deposition area may show a tapering along its width at the crotch region of the core. In this way, the absorbent material deposition area may have a relatively narrow width in an area of the core intended to be placed in the crotch region of the absorbent article. This may provide for example better wearing comfort.

The method of the invention is directed at characterizing channels which may be present in the absorbent core. The absorbent cores tested may thus comprise at least one channel, in particular at least two channels 26 (referred to herein as "channels" to include the singular and plural form) as illustrated in Fig. 1. The term "channel" designates a generally longitudinally extending area of the core comprising less absorbent material, or material that has been compressed, than the surrounding areas so that an insulting fluid can be quickly distributed along the channel towards the front and back of the core. The channels may also serve to facilitate the folding of the core in a desired configuration during wear. The channels may be substantially free of absorbent material. By "substantially free" it is meant that in each of the channel areas, the basis weight of the absorbent material is at least less than 25%, in particular less than 20%, less than 10%, of the average basis weight of the absorbent material in the rest of the absorbent material deposition area of the core. In particular the channels may be (substantially) free of absorbent material. Minimal amount such as involuntary contaminations with absorbent material particles that may occur during the making process are disregarded for the purpose of considering if there is absorbent material in the channels. The channels 26 are advantageously surrounded by the absorbent material 60, when considering the plane of the core, which means that the channels do not extend to any of the edges of the deposition area 8 of the absorbent material 60.

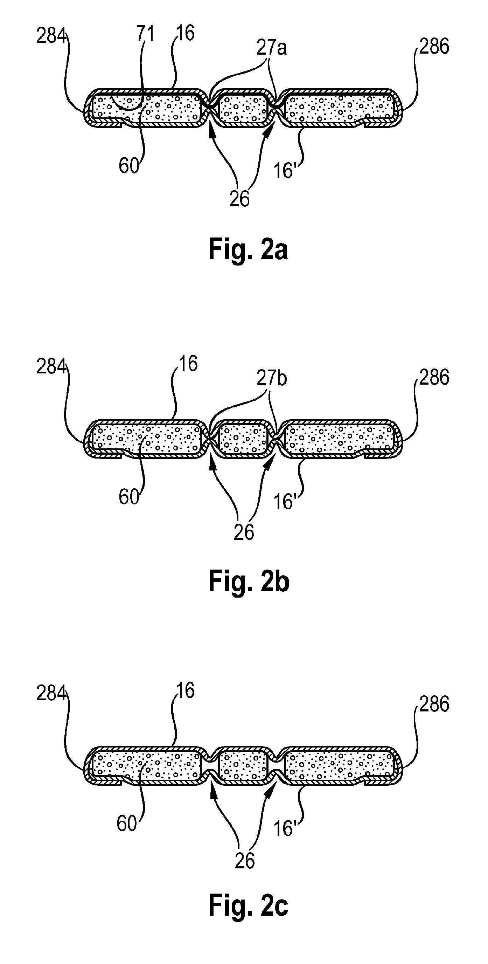

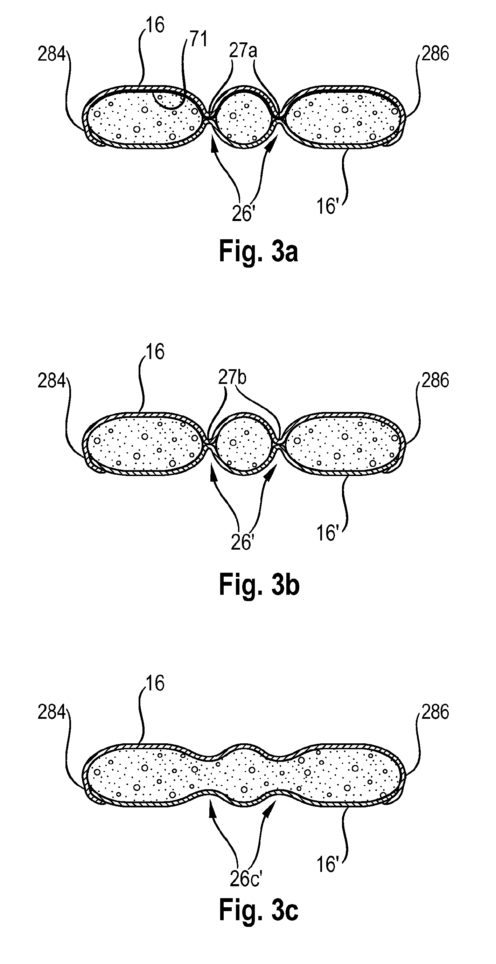

The top layer 16 and the bottom layer 16' of the core wrap maybe bonded to each other through these channels 26 by channel bonds 27. As will be detailed further below, the channel bonds 27 between the top layer and the bottom layer of the core wrap through the channels may be formed by any suitable methods known in the art. A first bonding method is to form a glue bond 27a (typically together with local compression in the channel areas to ensure good adherence of the adhesive) by an auxiliary glue 71 applied directly to one of the inner surface of core wrap as illustrated in Fig. 2a. Other bonding methods are possible such as locally applying pressure, heat or ultrasonic bonding and combination thereof, as illustrated by core wrap bond 27b in Fig. 2b. Channel bonds allows the channels 26 to form more pronounced three-dimensional channels 26' as the absorbent material swells when it absorbs a liquid such as urine as illustrated in Fig. 3a,b. Strong channel bonds are described in details for example in WO2012/170778 (Rosati et al.), WO2014/93311A1 (Arizti et al), WO2014/093310 (Ehrnsperger et al.) which disclose absorbent structures that comprise superabsorbent polymers, optionally a cellulosic material, and at least a pair of substantially longitudinally extending channels. Channel areas 26 substantially free of absorbent material can be formed for example by modifying the pattern of the grid and receiving drums so that no SAP is applied in the selected areas, as exemplary disclosed in US2012/0312491 and US2014/027066 (Jackels et al.).

The absorbent core may comprise at least a first channel and a second channel disposed on each side of the longitudinal axis 80, which longitudinally divides the absorbent core in two halves. It is not excluded that the core may also comprise more than two channels or only one channel. Shorter channels substantially free of absorbent material may also be present, for example in the back region or the front region of the core, as seen for example in the Figures of WO2012/170778.

The channels typically extend generally longitudinally, which means that each channel extends at least as much in the longitudinal direction (80) than in the transversal direction (90), and typically at least twice as much in the longitudinal direction than in the transverse direction (as measured after projection on the respective axis). The channels 26 may have a length L' projected on the longitudinal axis 80 of the core that is at least 10% of the length L of the absorbent core, in particular from 20% to 80%. The channels may have an area substantially free of absorbent material having a width Wc along at least part of their length which is at least 2 mm, or at least 3 mm or at least 4 mm, up to for example 20 mm, or 16 mm, or 12 mm. The width Wc may be constant through substantially the whole length or may vary along the length of the channels.

The channels 26 may be curved towards the longitudinal axis as shown in the Figures but they may be also straight and parallel to the longitudinal axis. There may be or may be no channels that coincide with the longitudinal axis 80 of the core. When present as a pair of channels disposed symmetrically relative to the longitudinal axis 80 of the core, these may be spaced apart from one another over their whole longitudinal dimension. The smallest spacing distance maybe for example at least 5 mm, or at least 10 mm, or at least 16 mm.

The exemplary core of Fig. 1 comprises a pair of longitudinally extending channels 26. Channels may be further characterized by the presence or absence of bonds between the top layer 16 of the core wrap and the bottom layer 16' of the core wrap in the channel areas. This is illustrated in the different views of Fig. 2a, 2b and 2c. Fig. 2a illustrates an absorbent core as in Fig. 1 with the top side and the bottom side of the core wrap permanently bonded to each other through the channels. Such a strong core wrap bond 27a may be for example obtained by gluing the core wrap to itself in the channel areas using an auxiliary glue 71, that may be present also outside the channel areas 26 to at least partially immobilize the absorbent material. Fig. 2b illustrates a similar core with channel bonds 27b but wherein the bond is weaker and will break upon moderate load and/or squeezing of the core as will be explained further below. The channel bonds 27b can be qualified to be semi-permanent. Such bonds may be for example formed by the local application of heat and/or pressure to locally melt the bottom and top layers. Finally Fig. 2c illustrates a similar absorbent core as in Fig. 1 comprising channels formed by absorbent material free areas but without bonding of the core wrap in the channel areas. This type of channel may be qualified to be unbounded or non-bonded.

Fig. 3a, b, c schematically illustrate the state of the channels of the cores of Fig. 2a, b, c after a moderate amount of fluid has been absorbed by the absorbent material. A moderate amount may correspond to a first insult by a fluid. Such an amount may for example correspond to from about 1/3 to 2/3 of the overall capacity of the absorbent material. As the absorbent core when dry may be very thin, especially when the absorbent material consists of superabsorbent polymer, the channels in the swollen core 26' become more pronounced and easy to be tactilely felt after the absorbent material surrounding them has swollen. On the other hand, when the channels were unbounded as in Fig. 3c, the surrounding swollen material may swell in and fills the areas of the channels 26'c, so that these non-bonded channels quickly stop operating.

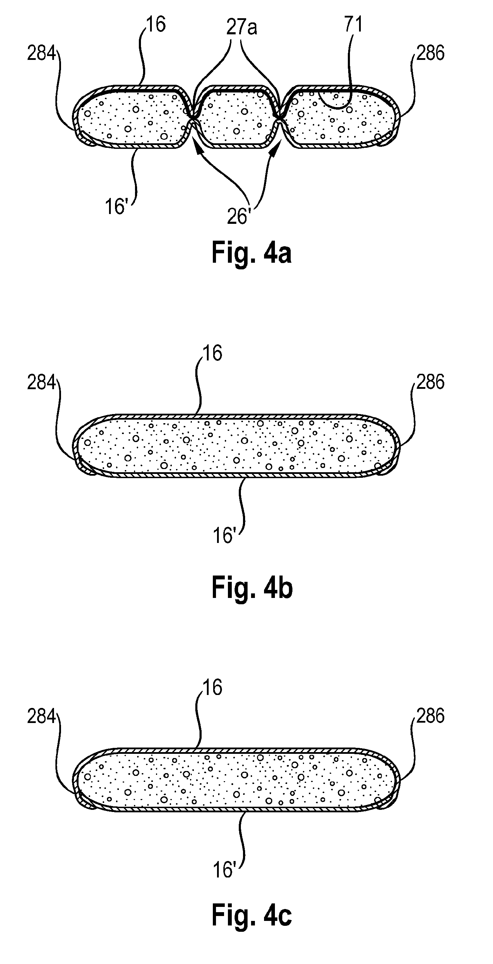

Fig. 4a, b, c illustrate the state of the channels of the cores of the previous Figures after the absorbent core has been subjected to a physical actions, for example squeezed, shaken or laterally compressed, and/or when the absorbent core has absorbed a further amount of fluid. These further treatments put additional strains on the channel bonds 27. The weaker channel bonds 27b as illustrated in Figs. 2b, 3b have not resisted this further handling so that they have completely released and the swollen absorbent material has filled the channel areas as in Fig. 4b. The absorbent core of Fig. 4c also no longer shows any signs of the previously present channels. Accordingly the channels of the core of Figs. 4b-4c can no longer perform any fluid distribution or folding function. On the other hand, the permanent channels of the core shown in Fig. 2a, 3a, 4a remain present and functional longer than the channels of the other cores. As indicated previously, even permanent bonds may be designed to break at a very large load to release space for the swelling absorbent material, however this may be designed to happen close to saturation, for example when the absorbent material as absorbed about more than 2/3 of its maximum capacity.

Although the prospective user of an absorbent article maybe aware of the presence of channels in the absorbent core through the manufacturer's product description, the user has however typically no possibilities to check the presence of bonds within the channel areas, and when these are present how strong and resilient these are. Absorbent articles for personal hygiene such as diapers or adult incontinence products are often worn under clothing, so that it is difficult for the user to assess during use if the channels are permanent, semi-permanent or un-bonded. Furthermore diapers worn for a long time, in particular overnight, may be close to saturation when they are changed so that even permanent bonds may have broken when they are inspected for change by the caregiver.

### Method of the invention

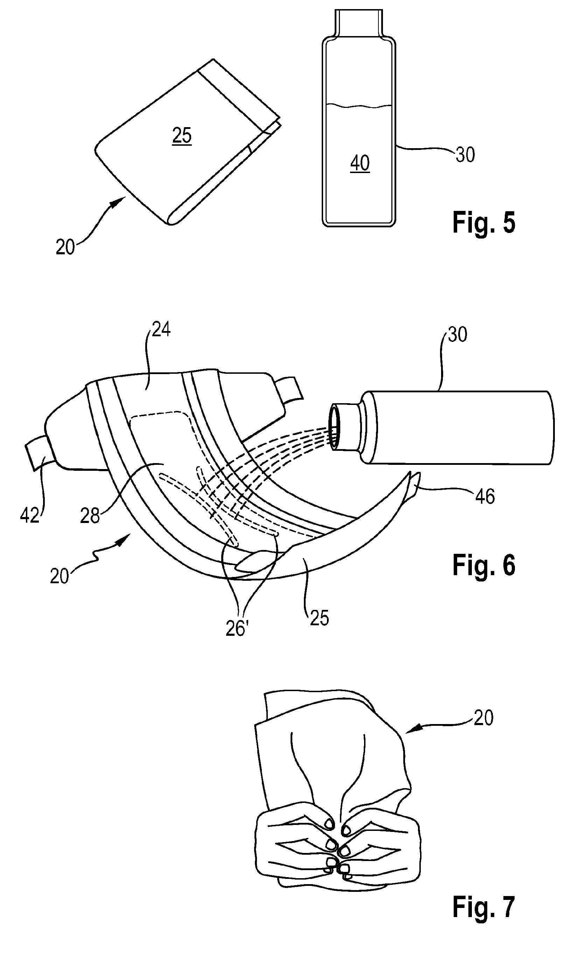

The method of the invention addresses this problem and provides a simple, cost-efficient and visual assessment for determining the presence of channel bonds, and if channel bonds are present the quality (i.e. their resiliency to core swelling / handling) of these channel bonds. The different method steps of the invention are illustrated in Figs. 5-9 with an absorbent core comprised in an absorbent article 20 in the form of a taped baby diaper absorbent. These are exemplary illustrations and should not be considered limiting as many variations are possible. As indicated previously, the absorbent core alone may be tested directly, but the method is also suitable for testing any absorbent articles such as pant-type diapers as well as a feminine care and/or adult incontinence products that contain such an absorbent core without the need to separate the absorbent core from the remaining layers of the article.

Fig. 5 illustrates the step of providing an absorbent core (comprised in an article 20) and a container 30 containing a test liquid 40. The method may be for example conducted by a manufacturer of absorbent articles to demonstrate the properties of the channels in its products, or by a potential user or independent tester wishing to test and compare different products. Diapers are typically sold bi-folded with the wearer-facing backsheet 25 forming the outside surface. Other components, such as the topsheet 24, the back ears 42, the front ears 46 and longitudinally extending barrier leg cuffs become visible once the article is un-folded as shown on Fig. 6. The topsheet 24, the backsheet 25, the absorbent core 28 and the other article components may be assembled in a variety of well-known configurations, in particular by gluing and/or heat embossing. Exemplary diaper assemblies are for example generally described in US3,860,003, US5,221,274, US5,554,145, US5,569,234, US5,580,411 and US6,004,306, articles having channels being more specifically disclosed for example in US2012/0316526A1, US7,850,672, and US7,888,549.

The liquid 40 provided may be any liquid that can be absorbed by the absorbent core. Typically an aqueous liquid will be provided. Tap water may for example be used by a tester which is a user at home without any further preparation for the liquid. A more sophisticated liquid may be also prepared, for example comprising a colorant to better highlight the swollen material. Typical colorants used for demonstration provide water with a blue or green color but any other color may also be used. The test liquid may also comprise some sodium chloride at the same level as urine for example. The liquid may be contained in any type of container. The container may be advantageously graduated to that the person conducting the test can control in a reproducible manner the amount of fluid dispensed on the article. At home, a tester may use for example a feeding-bottle with graduation.

Fig. 6 illustrates the step of pouring the liquid on the absorbent article/core so that the absorbent core absorbs at least some of the liquid. Advantageously, a pre-determined amount of liquid will be poured to ensure reproducibility, especially if two different products are to be tested and compared. If only a rough evaluation of the permanency of the channels is desired, the tester may also use a relatively imprecise amount of fluid (a small glass of water for example). The amount of liquid may be pre-determined, for example to simulate a first gush of urine from a baby, a liquid amount of about 75 ml may be used for a (Size 4) diaper (more generally between 50 ml and 100 ml), but for smaller or larger sizes other amount may be used (for example from 20 ml to 150 ml). The liquid may be typically poured towards the middle of the article/core representing the typical point of fluid insult. Typically, the three-dimensional channels 26' formed after the absorbent material surrounding the channel areas has swollen will already become clearly visible after this first application. A second amount of liquid may be poured after the first pouring of the liquid to simulate a second gush of urine, for example the same amount as previously poured, or more generally any amount of liquid. After the liquid has been absorbed, the tester may then gently touch the wearer-facing surface and the garment-facing surface of the article or absorbent core to feel the three-dimensional channels 26'. At this stage, it will be typically already visually and tactilely recognizable by the tester if the absorbent core comprised any type of channels. Furthermore, non-bonded channels, which do not comprise channel bonds, will start filling with the swollen absorbent material already at this stage.

In an optional but advantageous step, the tester may exert a physical action on the absorbent core/article before the step of cutting the absorbent core. The tester may for example press, shake or squeeze the diaper with moderate force as illustrated in Fig. 7 to simulate physical activities of a prospective wearer such a walking, jumping or sitting down. If a more reproducible and quantitative method is desired, one could place the absorbent core/article between two plates equipped with a sensor and a motor so that a predetermined lateral compressing force is reproducibly applied for a pre-determined amount of time. In most cases however, the pressure may be applied manually by the tester in a reliable manner. If the absorbent article tested is a taped diaper, the back ear tapes 42 maybe attached to the landing zone (not represented) at the front of the diaper on the garment-facing side to simulate the diaper being worn.

The test method further comprises the step of cutting the absorbent article/core in two halves. A sharp pair of scissors 50 or any other suitable cutting device can be used to cut through the materials of the tested product in the areas where the channels are present, if known. The absorbent article/core may be cut generally perpendicularly to the direction of the channels so that the cut goes through the channels 26'. The article/core maybe cut transversally towards its middle corresponding to the transversal axis 90. If the channels are skewed towards the front or back of the article/core, the tester may chose to cut accordingly closer to the longitudinal center of the channels than the center of the tested product. It may be recommended to use a disposable underlay during the whole process as some of the absorbent material may escape through the cut.

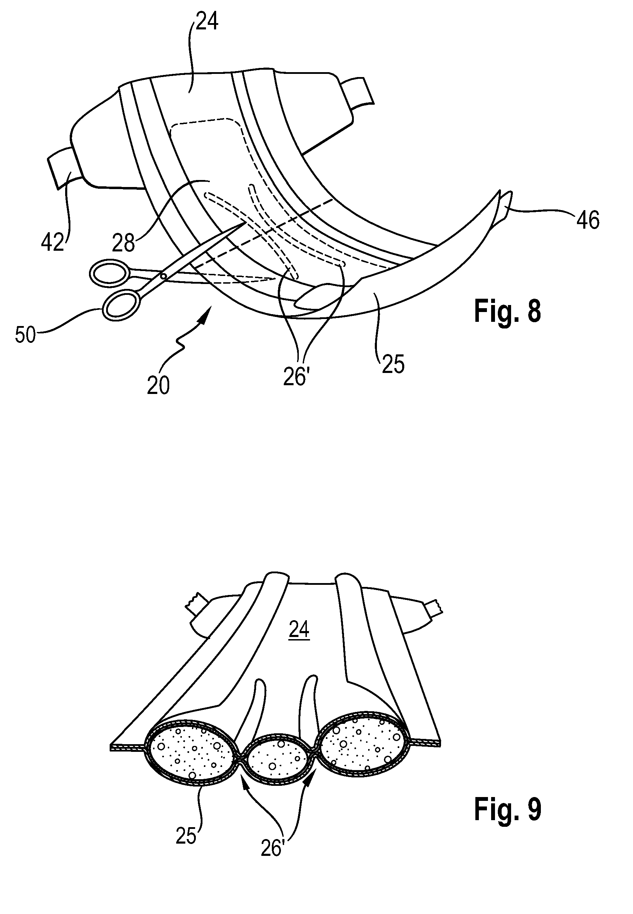

After cutting, the tester can separate the two halves of the cut core/article and visually assess the presence or absence of the channel bonds 27 in one half as illustrated in Fig. 9, or each of the two halves, as well as their quality, for example: present and strong (corresponding to permanent channels), present but weakened or broken (corresponding to semi-permanent channels), no longer present (corresponding to un-bonded channels). A photo may be taken to record the result and facilitate comparison with other products tested separately. In a variation of the sequence of the method steps discussed before, it is also possible to first cut the absorbent article/core and then pour a liquid onto one of the core/article half to better observe the swelling of the absorbent material and the dynamic evolution of the channels. It may be thus possible to see the non-bonded or weakly bonded channels rapidly opening and filling with absorbent material, whereas permanent channels will maintain their integrity as the absorbent material swells. This alternative also allows the tester to see how the liquid is pulled from the topsheet and then the active absorption of the fluid in the areas of the core between the channels when performed on an article. Careful handling of the cut core may be required as the dry absorbent material particles may more easily fall from the cut than swollen absorbent gelling material particles.

The method of the invention can be repeated with different absorbent articles/cores to compare the channel bond strength in different products, advantageously using the same conditions for each test. The present method is easy to perform by unskilled testers for qualitative testing but can also be used for quantitative testing by professional or trained testers. The method may also be used to demonstrate the presence of channels in an absorbent article/core and compare this result with a different absorbent article/core having no channels or channels which are unbounded or non-permanently bonded. Such a method for comparing a first absorbent core with a second absorbent core, may include the steps of:
- performing the method described previously on the first absorbent core to obtain a first result relative to the channel(s) of the first absorbent core;
- performing the steps of the method indicated above on the second absorbent core to obtain a second result relative to the presence and quality of the channel(s) of the second absorbent core if these are present, and the absence of channels if these are absent in the second absorbent core; wherein the first absorbent core and the second absorbent core are advantageously tested under similar conditions, and
- comparing the first result to the second result.

The first absorbent core may for example comprise at least one permanent channel bond extending in the longitudinal direction, in particular formed by gluing of the top layer of the core wrap to the bottom layer of the core wrap through the channels. The second absorbent core may not comprise any channels, or comprise channels which are un-bonded, or comprise channels which are less strongly bonded than the channels of the first absorbent core. As indicated previously, the first and second absorbent cores may be tested in isolation or integrated in ready-to-use absorbent articles.

The method of the invention has been described for testing channels in an absorbent core, but is also applicable to testing channels between other layers of an absorbent article, for example between the topsheet and the backsheet, or an acquisition/distribution layer and the topsheet, or an acquisition/distribution layer and the top layer of the core wrap, or between the bottom layer of the absorbent core and the backsheet.

## Claims

1. A method for evaluating the permanency of channel(s) in an absorbent core, the method comprising the steps of:
- providing an absorbent core (28), optionally in an absorbent article (20), extending in a longitudinal direction (80) and a transversal direction (90), wherein the absorbent core comprises a core wrap having a top layer (16) and a bottom layer (16'), and an absorbent layer (8) between the top layer and the bottom layer, wherein the absorbent layer comprises an absorbent material (60) and at least one channel (26), preferably at least two channels, extending generally in the longitudinal direction;
- providing a liquid (40);
- pouring the liquid into the absorbent core so that the absorbent layer absorbs at least some of the liquid;
- cutting the absorbent core in the transversal direction in an area of the core where the at least one channel is present; and
- evaluating the presence or absence of channel bonds (27) between the top layer and the bottom layer through the cut in the core, and if channel bonds are present optionally evaluating the quality of the channel bonds.

2. A method according to claim 1, wherein the pouring step takes place before the cutting step and the method further comprising the step of exerting a physical action on the absorbent core between the step of pouring the liquid into the core and the step of cutting the core.

3. A method according to claim 2, wherein the physical action is manually squeezing and/or shaking the absorbent core.

4. A method according to any of the preceding claims, wherein the step of pouring the liquid comprises a first pouring step wherein a first quantity of liquid is poured, and a second pouring step wherein a second quantity of liquid is poured.

5. A method according to the preceding claim, wherein the first quantity of liquid and the second quantity of liquid are about equal.

6. A method according to any of the preceding claims, wherein the liquid is an aqueous liquid comprising a colorant, especially when the colorant is blue.

7. A method according to any of the preceding claims, wherein the absorbent core comprises a first channel and a second channel disposed symmetrically relative to the longitudinal axis (80) of the core, and wherein the channels are straight and oriented parallel to the longitudinal direction, or the channels are concavely curved towards the longitudinal axis of the core.

8. A method according to any of the preceding claims, wherein the at least one channel is an area within the absorbent layer substantially free of absorbent material, and wherein the top layer of the core wrap is attached to the bottom layer of the core wrap by a core wrap bond (27) through the substantially absorbent material free channel.

9. A method according to the preceding claim, wherein the core wrap bond is formed by at least one method selected from gluing, pressure bonding, heat bonding and/or ultrasonic bonding.

10. A method according to any of the preceding claims, wherein the absorbent layer is substantially free of cellulose fibers.

11. A method according to any of the preceding claims, wherein the absorbent core is comprised in an absorbent article (20) having a topsheet (24) on a wearer-facing side, a backsheet (26) on a garment-facing side and the absorbent core is between the topsheet and the backsheet.

12. A method for comparing a first absorbent core with a second absorbent core, the first and second absorbent cores being optionally comprised in a first and second absorbent articles respectively, wherein the first absorbent core is according to claim 1 and the second absorbent core optionally comprises at least one channel, the method including the steps of:
- performing the method of claim 1 on the first absorbent core to obtain a first result relative to the presence or absence, and optionally the quality, of the channel bonds of the first absorbent core;
- performing the steps of the method of claim 1 on the second absorbent core to obtain a second result relative to the presence or absence, and optionally quality, of the channel bonds of the second absorbent core if these are present; wherein the first absorbent core and the second absorbent core are tested under similar conditions, and
- comparing the first result to the second result.

13. A method according to the preceding claim, wherein the first absorbent core comprises channel bonds formed by gluing of the top layer of the core wrap to the bottom layer of the core wrap through the at least one channel.

14. A method according to claim 12 or 13, wherein the second absorbent core before being tested does not comprise channels, or comprises channels which are un-bonded, or comprises channels which are less strongly bonded than the channels of the first absorbent core.

15. A method for evaluating the permanency of channels in an absorbent article, the method comprising the subsequent steps of:
- providing an absorbent article (20), extending in a longitudinal direction (80) and a transversal direction (90), and wherein the absorbent article comprises a least one channel (26), preferably at least two channels, generally extending in the longitudinal or transverse direction;
- providing a liquid (40);
- pouring the liquid into the absorbent article so that the absorbent article absorbs at least some of the liquid;
- cutting the absorbent article in a direction generally perpendicular to the channels; and
- evaluating the presence or absence of channel bonds (27), and if channel bonds are present optionally evaluating the quality of the channel bonds.
